# FASCICULE DE BREVET EUROPEEN

(11) **EP 3 897 395 B1**
(45) Date de publication et mention de la délivrance du brevet: **25.01.2023**
(21) Numéro de dépôt: 19835684.2
(22) Date de dépôt: 10.12.2019
(51) Int. Cl.: A61B 10/00, A61M 1/00

(54) **DISPOSITIF DE RECUEIL DE CALCULS URINAIRES**
VORRICHTUNG ZUM SAMMELN VON HARNSTEINEN
DEVICE FOR COLLECTING URINARY STONES

(30) Priorité: 17.12.2018 FR 1873026
(43) Date de publication de la demande: 27.10.2021
(73) Titulaire: Freestone, 13013 Marseille (FR)
(72) Inventeur: GIAIME, Philippe, 13011 Marseille (FR); SAMPOL, Jerome, 13007 Marseille (FR)
(74) Mandataire: Marchand, André
(86) Numéro de dépôt international: PCT/FR2019/000206
(87) Numéro de publication internationale: WO 2020/128168

(56) Documents cités:
- JP-A- H04 105 647
- US-A1- 2009 254 056
- US-A1- 2017 105 707
- US-A1- 2018 303 465
- US-B1- 6 389 609
- US-B1- 6 651 259

## Description

La présente invention concerne un dispositif pour collecter des calculs urinaires.

De tels calculs urinaires sont à l'origine de colique néphrétique. Cette maladie extrêmement répandue et qui tend à s'étendre, est due au blocage d'un calcul dans les voies urinaires. Il en résulte un défaut de drainage, ce qui provoque une mise en tension des cavités rénales, et une douleur très intense. Il s'avère que la formation de calculs est plus fréquente chez les patients obèses, ainsi que chez les patients diabétiques.

Par ailleurs, la nature des calculs peut varier d'un patient à l'autre. Ainsi, les calculs d'oxalate de calcium et d'acide urique sont plus fréquents chez les hommes, tandis que les calculs de phosphate de calcium sont plus fréquents chez les femmes.

Il s'avère également que le taux de récidive de l'apparition de calculs est très élevé, de l'ordre de 50% en France, après une période moyenne de 3,5 ans entre deux apparitions de calculs. Il est donc essentiel de tenter d'éviter la récidive et donc de prévenir l'apparition de calculs rénaux et/ou urinaires. A cet effet, plusieurs protocoles de traitement existent, mais leur définition dépend de la composition des calculs. Or la composition des calculs ne peut pas être déterminée avec certitude par d'autres moyens tels qu'une analyse de sang ou d'urine. Il est donc nécessaire de collecter les calculs en vue de leur analyse directe par spectrophotométrie.

Actuellement, il est demandé aux patients d'utiliser un filtre en papier (par exemple du type filtre à café) ou une compresse en tissu pour collecter les calculs urinaires. Ces procédures s'avèrent peu pratiques, et peu hygiéniques, mettant les doigts des patients en contact avec l'urine, notamment pour récupérer le calcul dans le filtre ou la compresse. Par ailleurs, le filtre en papier peut se déchirer ou se renverser durant la collecte, avec pour conséquence la perte du calcul. Ces dispositifs sont à usage unique. Par conséquent, le patient doit avoir à sa disposition plusieurs filtres ou compresses pour pouvoir effectuer plusieurs tentatives de collecte pendant une journée. En pratique, très peu de calculs urinaires sont ainsi collectés (moins de 15%).

Il s'avère également que la connaissance du pH (potentiel Hydrogène) urinaire au moment de la collecte du calcul est déterminante pour permettre l'obtention d'un diagnostic fiable.

Le brevet US2896788 décrit un dispositif en papier rigide, pliable, en forme de conduit pyramidal, et comprenant un volet interne formant un filtre, qui peut être rabattu pour obturer le conduit. Ce dispositif impose au patient de récupérer le calcul sur le filtre pour le disposer par exemple dans une poche étanche, pour l'envoyer à un laboratoire d'analyse. Après usage, le dispositif se trouve imprégné d'urine et doit donc être jeté.

Le brevet US5762071 décrit un dispositif de collecte de calculs urinaires comprenant un corps cylindrique, dans lequel est disposé un filtre, et deux couvercles adaptés pour se visser sur les deux ouvertures opposées du corps cylindrique. Une fois refermé des deux côtés, ce dispositif peut être transmis à un laboratoire d'analyse. Ce dispositif s'avère peu pratique en raison de la présence de trois pièces séparées, et de la nécessité de dévisser et de visser les deux couvercles à chaque tentative de collecte.

La demande de brevet JP H04 105647 décrit un dispositif de collecte de calculs urinaires comprenant un filtre, un couvercle lié au filtre par une articulation, pour retenir des calculs urinaires collectés par le filtre en position fermée, et servant de poignée en position ouverte. D'autres dispositifs de collecte de calculs urinaires sont décrits dans les demandes de brevet US 2017/105707 et US 2009/254056, et les brevets US 6 651 259 et US 6 389 609.

Il est donc souhaitable de proposer un dispositif de collecte de calculs urinaires qui soit facile à utiliser aussi bien par les femmes que les hommes. Un tel dispositif de collecte devrait également permettre de maintenir les calculs recueillis, tout en assurant une certaine hygiène en n'obligeant pas le patient à mettre ses doigts en contact avec l'urine. Il peut être également souhaitable que le dispositif de collecte puisse être utilisé plusieurs fois, jusqu'à ce que des calculs soient collectés, et que les calculs collectés puissent être transmis à un laboratoire d'analyse sans avoir besoin d'être manipulés. Il peut être également souhaitable de permettre la réalisation par le patient d'une analyse du pH urinaire.

L'invention concerne un dispositif de collecte de calculs urinaires comprenant : une partie filtre présentant une face concave et une face opposée convexe, et une multiplicité de trous formant un filtre, une partie couvercle liée à la partie filtre par une articulation, de manière à être mobile entre une position déployée où elle assure une fonction de poignée, et une position fermée où elle couvre la face concave de la partie filtre et ferme un volume entre les parties filtre et couvercle, pour retenir des calculs urinaires collectés par le filtre, et un dispositif de blocage des parties filtre et couvercle en position fermée. Selon l'invention, la partie filtre comprend une cuvette pour recueillir de l'urine pour effectuer une analyse de pH urinaire.

Selon un mode de réalisation, l'articulation présente un axe d'articulation orienté parallèlement à un plan d'ouverture de la partie filtre.

Selon un mode de réalisation, la partie couvercle présente une forme générale identique à la partie filtre.

Selon un mode de réalisation, l'articulation présente un axe d'articulation orienté perpendiculairement à un plan d'ouverture de la partie filtre.

Selon un mode de réalisation, la partie couvercle présente une face concave et une face convexe, la face convexe du couvercle étant disposée en position fermée en regard de la partie filtre, de manière à assurer le blocage des parties filtre et couvercle en position fermée.

Selon un mode de réalisation, le dispositif comprend un volet couvrant la face concave de la partie filtre, le volet étant lié par une articulation à la partie filtre et/ou à la partie couvercle, de manière à être mobile entre une position fermée où il coopère avec la partie couvercle pour former un volume fermé logeant la partie filtre, et une position déployée où il coopère avec la partie couvercle pour assurer une fonction une poignée de tenue de la partie filtre.

Selon un mode de réalisation, la partie couvercle comprend deux parties latérales liées de manière pivotante à la partie filtre, afin d'être mobiles entre une position fermée où elles forment un volume fermé logeant la partie filtre, et une position déployée où elles coopèrent ensemble pour former une poignée de tenue de la partie filtre.

Selon un mode de réalisation, la partie couvercle forme un logement pour recevoir un rouleau de bandelettes de test de pH urinaire.

Des modes de réalisation peuvent également concerner un ensemble de collecte de calculs urinaires comprenant : un dispositif de collecte tel que précédemment défini, des bandelettes de test de pH urinaire, une fiche d'identification et de consignation d'un résultat de test de pH urinaire, et une enveloppe pour la transmission du dispositif de collecte et de la fiche d'identification et de consignation.

Selon un mode de réalisation, l'ensemble comprend une poche avec une fermeture pour recevoir et renfermer le dispositif de collecte de manière étanche à l'eau.

Des exemples de réalisation de l'invention seront décrits dans ce qui suit, à titre non limitatif en relation avec les figures jointes parmi lesquelles :
les figures 1A, 1B sont des vues en perspective, respectivement de faces inférieure et supérieure d'un dispositif de collecte de calculs urinaires en configuration déployée, selon un mode de réalisation,
la figure 2 est une vue de profil du dispositif de collecte de la figure 1A, en configuration déployée,
la figure 3 est une vue en perspective de la face inférieure du dispositif de collecte de la figure 1A, en configuration fermée,
la figure 4 est une vue de profil du dispositif de collecte de la figure 1A, en configuration fermée,
les figures 5A, 5b sont des vues en perspective, respectivement des faces extérieures et intérieures d'un dispositif de collecte de calculs urinaires en configuration déployée, selon un autre mode de réalisation,
la figure 6 est une vue de profil du dispositif de collecte de la figure 5A, en configuration déployée,
les figures 7A, 7B sont des vues en perspective, respectivement des faces inférieure et supérieure du dispositif de collecte de la figure 5A, en configuration fermée,
les figures 8A, 8B sont des vues en perspective, respectivement de faces supérieure et inférieure d'un dispositif de collecte de calculs urinaires en configuration déployée, selon un autre mode de réalisation,
la figure 9 est une vue en coupe longitudinale du dispositif de collecte de la figure 8A, en configuration déployée,
les figures 10A, 10B sont des vues en perspective, respectivement des faces inférieure et supérieure du dispositif de collecte de la figure 8A, en configuration fermée,
la figure 11 est une vue en coupe longitudinale du dispositif de collecte de la figure 8A, en configuration fermée.

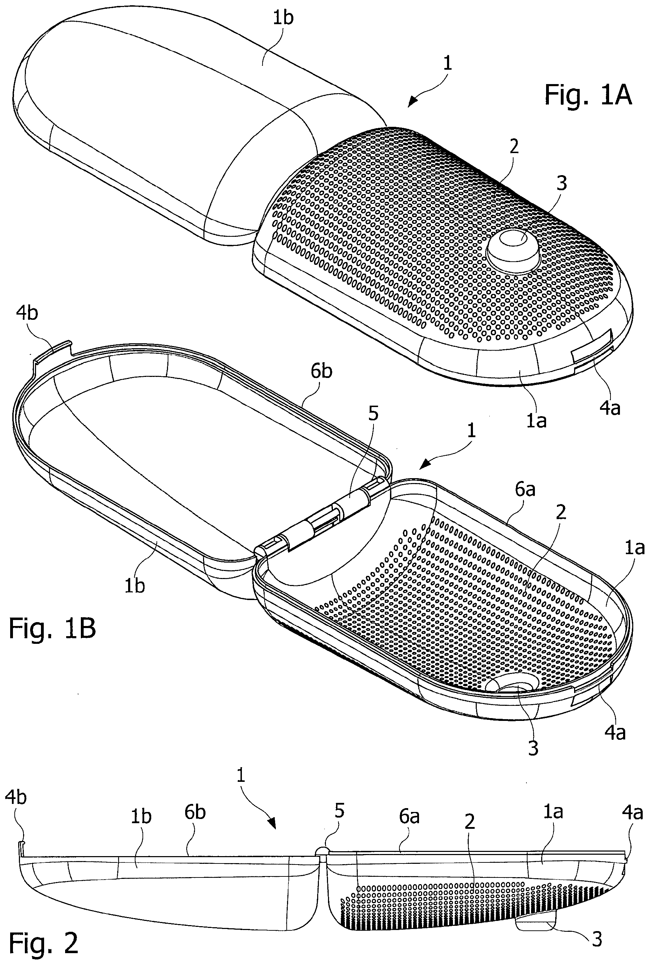

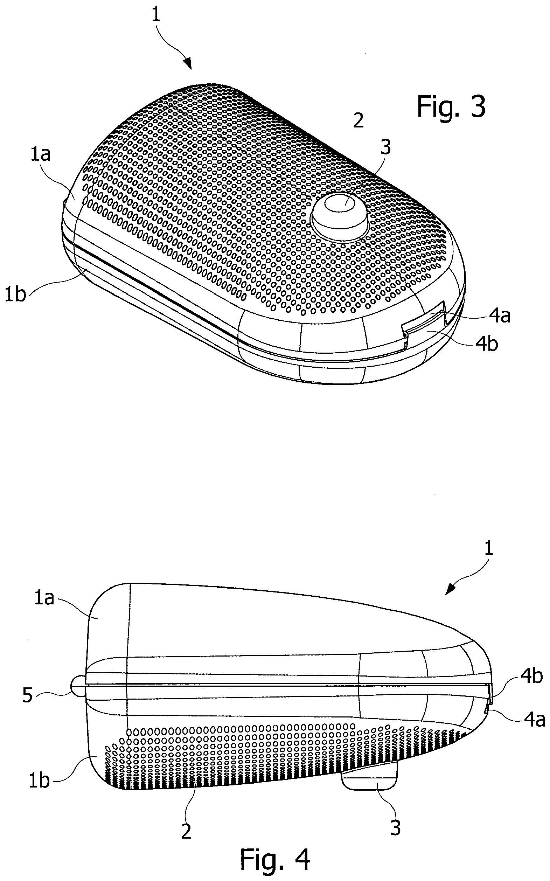

Les figures 1A, 1B, 2, 3, 4 représentent un dispositif 1 de collecte de calculs urinaires, selon un mode de réalisation. Le dispositif 1 comprend deux parties 1a, 1b en forme de demi-coquilles, à savoir une partie filtre 1a et une partie couvercle 1b. Chaque partie 1a, 1b présente une face externe concave et une face interne convexe. La partie filtre 1a comporte une multiplicité de trous de manière à former un filtre 2. La largeur des trous est adaptée à retenir des calculs urinaires d'un ou plusieurs millimètres de large. La répartition et le nombre de trous sont adaptés pour éviter tout risque d'accumulation d'urine dans la partie filtre. Les parties 1a, 1b sont liées l'une à l'autre par une articulation 5, de manière à pouvoir pivoter entre une position déployée montrée sur les figures 1A, 1B, et 2 et une position fermée montrée sur les figures 3 et 4.

Dans la position déployée, les parties 1a, 1b peuvent être agencées dans le prolongement l'une de l'autre, de part et d'autre de l'articulation 5, avec leurs faces concaves respectives du même côté. Dans cette position, la partie couvercle 1b forme une poignée ou un manche permettant de tenir le dispositif 1 dans une position adéquate pour recueillir des calculs urinaires. Les dimensions des parties 1a, 1b sont adaptées pour permettre aussi bien à une femme qu'à un homme d'uriner au travers du filtre 2. Dans la position fermée, les parties 1a, 1b délimitent un volume fermé, de manière à pouvoir retenir des calculs urinaires recueillis sur la partie filtre 1a. Ainsi, la partie 1b forme un couvercle adapté à la forme de la partie filtre 1a.

La partie filtre 1a comporte un dispositif de verrouillage 4a en position fermée, agencé de manière à coopérer avec un dispositif de verrouillage 4b de forme complémentaire prévu sur la partie couvercle 1b. L'articulation 5 présente un axe de pivotement situé dans un plan parallèle à des plans d'ouverture des parties 1a, 1b. Les moyens de verrouillage 4a, 4b sont disposés sur le bord 6a, 6b des parties 1a, 1b à l'opposé de l'articulation 5. Les bords 6a, 6b des pièces 1a, 1b peuvent être agencés de manière à ce qu'en position fermée, le bord (6a dans l'exemple de la figure 1B) de l'une des pièces (1a) se trouve à l'intérieur du bord (6b) de l'autre pièce (1b).

La partie filtre 1a peut présenter une forme sensiblement ovale dans un plan d'ouverture, avec une partie de bord rectiligne côté articulation 5. Elle peut être plus profonde du côté de l'articulation 5 que du côté du dispositif de verrouillage 4a. La partie couvercle 1b peut présenter une forme identique à la partie filtre 1a. Cependant, il n'est pas nécessaire que la partie couvercle 1b soit aussi profonde que la partie filtre 1a, puisqu'elle sert simplement de poignée ou de manche en position déployée et de couvercle en position fermée.

Selon un mode de réalisation, la partie filtre 1a comporte également une cuvette 3 agencée dans la région du filtre 2, de manière à pouvoir retenir un peu d'urine, en vue de permettre une analyse du pH urinaire, par exemple à la suite du recueil de calculs urinaires. La cuvette 3 peut être disposée le long d'un axe longitudinal de la partie filtre 1a, dans une région située entre la moitié et les 3/4 de la partie filtre en partant de l'axe de l'articulation 5.

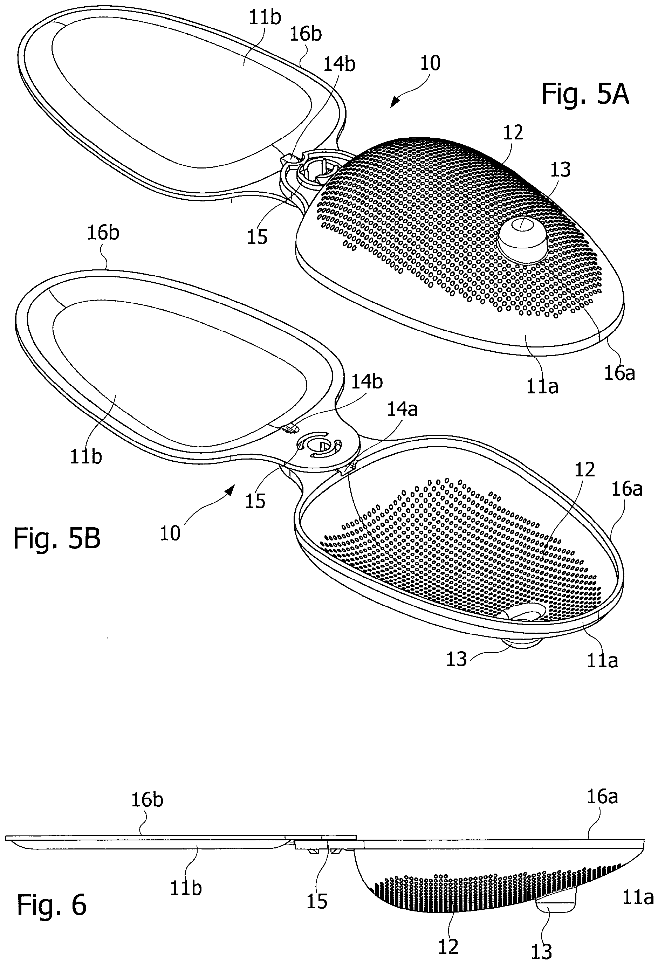

Les figures 5A, 5B, 6, 7A, 7B représentent un dispositif 10 de collecte de calculs urinaires, selon un autre mode de réalisation. Le dispositif 10 diffère du dispositif 1 principalement en ce que la partie filtre 11a et la partie couvercle 11b du dispositif 10 sont liées l'une à l'autre par une articulation 15 pivotante autour d'un axe perpendiculaire au plan du bord 16a de l'ouverture de la partie filtre 11a. Ainsi, les bords 16a, 16b des parties filtre 11a et couvercle 11b restent dans un même plan durant leurs déplacements relatifs entre les positions fermée et déployée. La partie couvercle 11b est sensiblement plane ou présente une face intérieure légèrement convexe et une face extérieure correspondante légèrement concave, pour s'engager dans la partie filtre 11a en position fermée.

La partie filtre 11a comprend une multiplicité de trous répartis de manière à former un filtre 12, et peut comprendre une cuvette 13 pour retenir un peu d'urine permettant d'effectuer un test de pH urinaire.

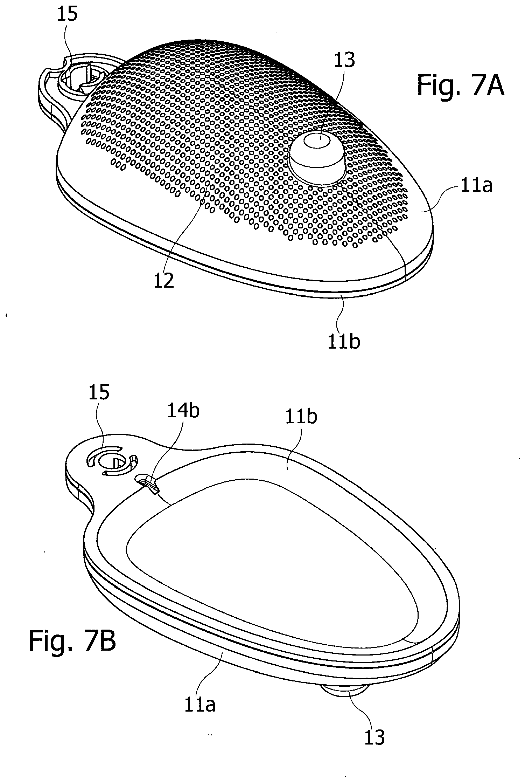

Pour maintenir le dispositif 10 en configuration fermée, un crantage est agencé sur des parties de bord en regard des parties 11a, 11b. Le crantage comprend à proximité de l'articulation 15 une bosse 14b agencée sur l'une des parties 11a, 11b pour s'engager en position fermée dans un creux 14a agencé sur l'autre partie 11a, 11b. Dans l'exemple des figures 5A, 5B, la bosse 14b est formée dans la partie couvercle 11b et le creux 14b est formé dans la partie filtre 11a. Il est à noter que le crantage 14a, 14b peut être omis, en particulier si la partie couvercle 11b présente une face convexe qui s'engage en position fermée dans la partie filtre 11a, comme cela apparaît en particulier sur les figures 6 et 7B.

Selon un mode de réalisation, il est prévu de renfermer totalement la partie filtre, afin que, notamment après une mise en contact avec de l'urine, elle puisse être complètement isolée dans un volume fermé. A cet effet, le dispositif comprend une partie inférieure montée mobile entre une position fermée où elle recouvre la face inférieure convexe (externe) de la partie filtre et se verrouille avec celle-ci ou bien avec la partie couvercle qui recouvre la face concave supérieure (interne) de la partie filtre, et une position déployée ou elle coopère avec la partie couvercle pour former un manche ou une poignée permettant de tenir le dispositif lors du recueil de calculs urinaires.

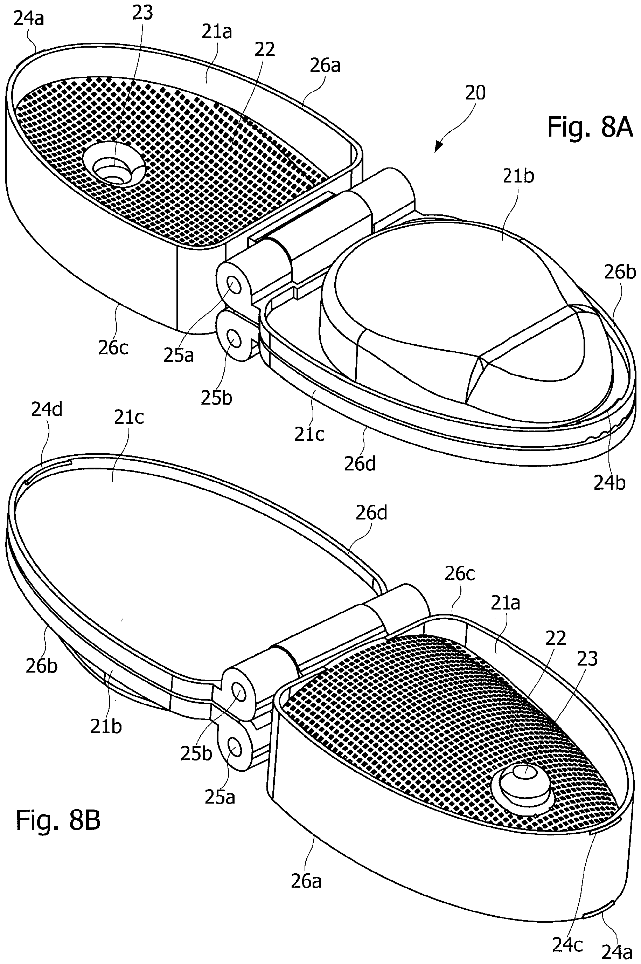

Les figures 8A, 8B, 9, 10A, 10B, 11 représentent un dispositif 20 de collecte de calculs urinaires, selon un autre mode de réalisation. Le dispositif 20 diffère du dispositif 1 principalement en ce qu'il comprend un volet inférieur 21c configuré pour recouvrir la face inférieure d'un filtre 22 et se verrouiller en position fermée sur une partie filtre 21a, le volet inférieur 21c étant lié à la partie filtre 21a par une articulation 25b. La partie filtre 21a présente une forme tubulaire ouverte à ses deux extrémités délimitées par des bords supérieur 26a, et inférieur 26c. Le passage entre les deux extrémités de la partie filtre 21a est fermé par le filtre 22 de forme bombée, présentant une face supérieure concave et une face inférieure convexe. Le filtre 22 peut comporter une cuvette 23 permettant de retenir un peu d'urine pour effectuer un test de pH urinaire. Le dispositif 20 comprend une partie couvercle 21b liée à la partie filtre 21a par une articulation 25a.

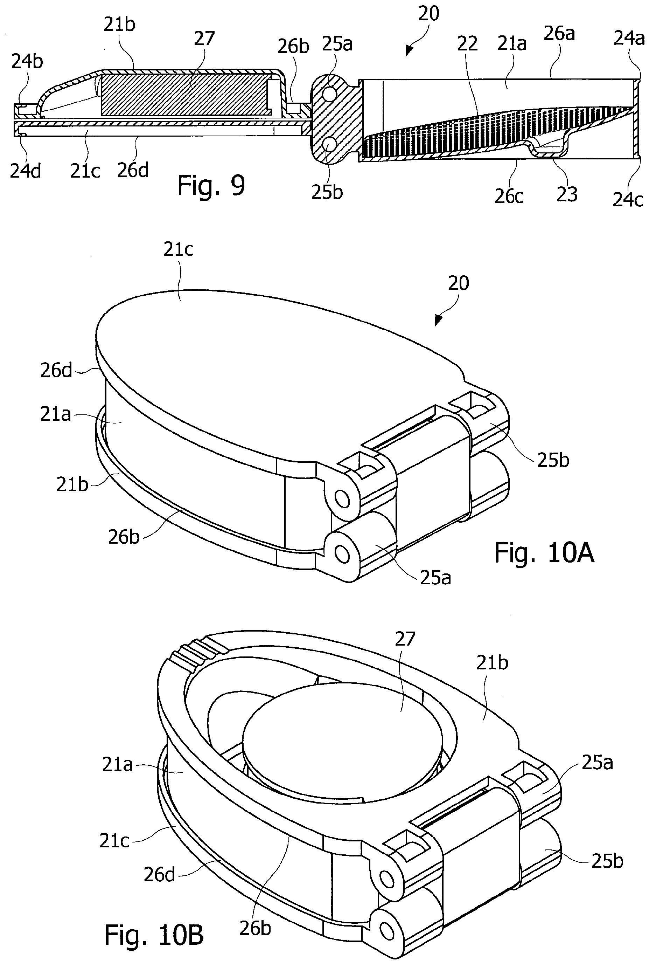

Les articulations 25a, 25b sont configurées pour qu'en configuration déployée (Figures 8A, 8B, 9), la partie couvercle 21a et le volet 21c puissent être placés l'un contre l'autre de manière à pouvoir former une poignée ou un manche apte à être tenu d'une seule main. Dans l'exemple des figures 8A, 8B, 9, 10A, 10B, 11, les axes des articulations 25a, 25b s'étendent dans un plan parallèle aux plans des bords 26a, 26c de la partie filtre 21a.

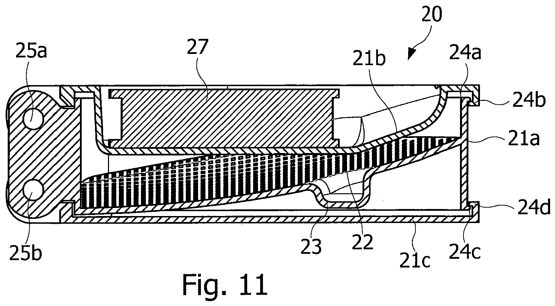

La partie filtre 21a comporte des dispositifs de verrouillage 24a, 24c en position fermée, agencés de manière à coopérer respectivement avec des dispositifs de verrouillage 24b, 24d de forme complémentaire prévus respectivement sur le bord 26b de la partie couvercle 21b et sur le bord 26d du volet 21c. Les dispositifs de verrouillage 24a-24d sont disposés sur une partie des bords 26a-26d des parties 21a, 21b et 21c à l'opposé des articulations 25a, 26b. Les bords 26a-26d des pièces 21a, 21b, 21c peuvent être agencés de manière à ce qu'en position fermée, des bords (26a, 26c en particulier sur la figure 11) se trouvent à l'intérieur des bords en regard (26b, 26d).

Selon un mode de réalisation, la partie couvercle 21a comporte une face externe (supérieure en position fermée de la partie couvercle) formant un logement prévu pour recevoir un rouleau 27 de bandelettes de test de pH urinaire (Figures 9, 10B, 11).

Selon un mode de réalisation, le dispositif 1, 10, 20 est entièrement réalisé par moulage ou impression 3D dans une matière plastique. Il présente une longueur comprise entre 8 et 12 cm, une largeur comprise entre 5 et 7 cm, et des profondeurs comprises entre 1,5 et 5 cm. Les trous formant le filtre 2, 12, 22 présentent une largeur comprise entre 0,5 et 1 mm. La cuvette 3, 13, 23 peut présenter une largeur et une profondeur comprises entre 0,8 et 1,5 cm.

Le dispositif 1, 10, 20 peut être distribué en association avec une poche étanche dans lequel le patient peut placer le dispositif 1 après usage, des bandelettes de test de pH urinaire qu'il peut utiliser avec l'urine retenue dans la cuvette 3, et une fiche sur laquelle il peut inscrire des informations d'identification et le résultat du test de pH urinaire

L'articulation 5, 25a, 25b peut être réalisée de différentes manières. Ainsi, dans le cas d'une articulation parallèle au plan de l'ouverture de la partie filtre, l'articulation peut être simplement réalisée à l'aide d'une membrane flexible solidaire des pièces 1a et 1b (ou 21a et 21b, ou 21a et 21c), par exemple dans le même matériau moulé en une seule pièce incluant les deux pièces. Dans ce cas, le dispositif 1 (ou les deux pièces 21a, 21b ou 21a, 21c) peut être réalisé en une seule pièce.

Selon un autre mode de réalisation, l'articulation 5, 25a, 25b est réalisée sous la forme d'un arbre cylindrique solidaire d'une des deux pièces 1a et 1b, (ou 21a et 21b, et/ou 21a et 21c), pivotant dans des gorges de forme cylindrique, solidaires de l'autre des deux pièces.

Il apparaîtra clairement à l'homme de l'art que la présente invention est susceptible de diverses variantes de réalisation. En particulier, l'invention n'est pas limitée aux modes de réalisation précédemment décrits et représentés dans les figures. Ainsi, dans tous les modes de réalisation précédemment décrits, l'axe de l'articulation peut être parallèle ou perpendiculaire au plan de l'ouverture de la partie filtre. Lorsque l'axe d'articulation est perpendiculaire au plan de l'ouverture de la partie filtre, comme dans l'exemple représenté sur les figures 5A, 5B, il peut être envisagé de jouer sur la souplesse du matériau formant le dispositif pour faire passer la face concave de la partie couvercle 11b (et/ou du volet 21c) entre ses positions fermée et déployée.

Selon un autre mode de réalisation, la partie couvercle est réalisée en deux parties latérales par exemple en forme de demi-coquilles, fixées de manière pivotante à la partie filtre suivant un axe perpendiculaire au plan de l'ouverture de la partie filtre, afin d'être mobiles entre une position fermée où elles renferment un volume fermé incluant la partie filtre, et une position déployée où elles forment ensemble une poignée de tenue de la partie filtre.

Par ailleurs, le filtre 2, 12, 22 peut être réalisé sous la forme de trous alignés en lignes et/ou en colonne, ou bien sous la forme d'un quadrillage formé de deux ensembles de nervures parallèles, les nervures d'un des ensembles ayant des orientations différentes des nervures de l'autre ensemble.

Il peut être également observé que les bords des parties filtre et couvercle ne sont pas nécessairement plans. En revanche, ils présentent des formes complémentaires pour assurer une fermeture complète du volume délimité par les parties filtre et couvercle, afin de pouvoir retenir les calculs pouvant être collectés par la partie filtre.

## Revendications

1. Dispositif de collecte de calculs urinaires comprenant :
une partie filtre (1a, 11a, 21a) présentant une face concave et une face opposée convexe, et une multiplicité de trous formant un filtre (2, 12, 22),
une partie couvercle (1b, 11b, 21b) liée à la partie filtre par une articulation (5, 15, 25a), de manière à être mobile entre une position déployée où elle assure une fonction de poignée, et une position fermée où elle couvre la face concave de la partie filtre et ferme un volume entre les parties filtre et couvercle, pour retenir des calculs urinaires collectés par le filtre, et
un dispositif de blocage (4a, 4b, 14a, 14b, 24a, 24b) des parties filtre et couvercle en position fermée,
**caractérisé en ce que** la partie filtre (1a, 11a, 21a) comprend une cuvette (3, 13, 23) pour recueillir de l'urine pour effectuer une analyse de pH urinaire.

2. Dispositif selon la revendication 1, dans lequel l'articulation (5, 25a) présente un axe d'articulation orienté parallèlement à un plan d'ouverture de la partie filtre (1a, 21a).

3. Dispositif selon l'une des revendications 1 et 2, dans lequel la partie couvercle (1b, 11b, 21b) présente une forme générale identique à la partie filtre (1a, 11a, 21a).

4. Dispositif selon la revendication 1, dans lequel l'articulation (15) présente un axe d'articulation orienté perpendiculairement à un plan d'ouverture de la partie filtre (11a).

5. Dispositif selon la revendication 4, dans lequel la partie couvercle (1b, 11b, 21b) présente une face concave et une face convexe, la face convexe du couvercle étant disposée en position fermée en regard de la partie filtre (1a, 11a, 21a), de manière à assurer le blocage des parties filtre et couvercle en position fermée.

6. Dispositif selon l'une des revendications 1 à 5, comprenant un volet (21c) couvrant la face concave de la partie filtre (21a), le volet étant lié par une articulation (25b) à la partie filtre et/ou à la partie couvercle (21b), de manière à être mobile entre une position fermée où il coopère avec la partie couvercle pour former un volume fermé logeant la partie filtre, et une position déployée où il coopère avec la partie couvercle pour assurer une fonction une poignée de tenue de la partie filtre.

7. Dispositif selon la revendication 1 à 5, dans lequel la partie couvercle comprend deux parties latérales liées de manière pivotante à la partie filtre, afin d'être mobiles entre une position fermée où elles forment un volume fermé logeant la partie filtre, et une position déployée où elles coopèrent ensemble pour former une poignée de tenue de la partie filtre.

8. Dispositif selon la revendication 1 à 7, dans lequel la partie couvercle (21b) forme un logement pour recevoir un rouleau (27) de bandelettes de test de pH urinaire.

9. Ensemble de collecte de calculs urinaires comprenant :
un dispositif de collecte selon l'une des revendications 1 à 8,
des bandelettes de test de pH urinaire,
une fiche d'identification et de consignation d'un résultat de test de pH urinaire, et
une enveloppe pour la transmission du dispositif de collecte et de la fiche d'identification et de consignation.

10. Ensemble selon la revendication 9, comprenant une poche avec une fermeture pour recevoir et renfermer le dispositif de collecte de manière étanche à l'eau.

## Patentansprüche

1. Vorrichtung zum Sammeln von Harnsteinen, umfassend:
einen Filterteil (1a, 11a, 21a), der eine konkave Seite und eine konvexe entgegengesetzte Seite und eine Vielzahl von Löchern aufweist, die einen Filter (2, 12, 22) ausbilden,
einen Deckelteil (1b, 11b, 21b), der mit dem Filterteil durch ein Gelenk (5, 15, 25a) verbunden ist, um zwischen einer ausgeklappten Position, in der er eine Grifffunktion gewährleistet, und einer geschlossenen Position bewegbar zu sein, in der er die konkave Seite des Filterteils abdeckt und ein Volumen zwischen dem Filter- und dem Deckelteil schließt, zum Zurückhalten der Harnsteine, die durch den Filter gesammelt werden, und
eine Verriegelungsvorrichtung (4a, 4b, 14a, 14b, 24a, 24b) des Filter- und des Deckelteils in geschlossener Position,
**dadurch gekennzeichnet, dass** der Filterteil (1a, 11a, 21a) eine kugelförmige Schale (3, 13, 23) zum Abfangen von Urin zum Durchführen einer Urin-pH-Wert-Analyse umfasst.

2. Vorrichtung nach Anspruch 1, wobei das Gelenk (5, 25a) eine Gelenkachse aufweist, die parallel zu einer Öffnungsebene des Filterteils (1a, 21a) ausgerichtet ist.

3. Vorrichtung nach einem der Ansprüche 1 und 2, wobei der Deckelteil (1b, 11b, 21b) eine allgemeine Form aufweist, die identisch mit dem Filterteil (1a, 11a, 21a) ist.

4. Vorrichtung nach Anspruch 1, wobei das Gelenk (15) eine Gelenkachse aufweist, die senkrecht zu einer Öffnungsebene des Filterteils (11a) ausgerichtet ist.

5. Vorrichtung nach Anspruch 4, wobei der Deckelteil (1b, 11b, 21b) eine konkave Seite und eine konvexe Seite aufweist, wobei die konvexe Seite des Deckels im Vergleich zu dem Filterteil (1a, 11a, 21a) in der geschlossenen Position angeordnet ist, um die Verriegelung des Filter- und des Deckelteils in der geschlossenen Position zu gewährleisten.

6. Vorrichtung nach einem der Ansprüche 1 bis 5, umfassend eine Klappe (21c), die die konkave Seite des Filterteils (21a) bedeckt, wobei die Klappe durch ein Gelenk (25b) mit dem Filterteil und/oder mit dem Deckelteil (21b) verbunden ist, um zwischen einer geschlossenen Position, in der sie mit dem Deckelteil zum Ausbilden eines geschlossenen Volumens zusammenwirkt, das den Filterteil aufnimmt, und einer ausgeklappten Position bewegbar zu sein, in der sie mit dem Deckelteil zum Gewährleisten einer Haltegrifffunktion des Filterteils zusammenwirkt.

7. Vorrichtung nach Anspruch 1 bis 5, wobei der Deckelteil zwei seitliche Teile umfasst, die mit dem Filterteil auf schwenkbare Weise verbunden sind, um zwischen einer geschlossenen Position, in der sie ein geschlossenes Volumen ausbilden, das den Filterteil aufnimmt, und einer ausgeklappten Position bewegbar zu sein, in der sie zum Ausbilden eines Haltegriffs des Filterteils zusammenwirken.

8. Vorrichtung nach Anspruch 1 bis 7, wobei der Deckelteil (21b) ein Gehäuse zum Empfangen einer Rolle (27) von Urin-pH-Wert-Teststreifen ausbildet.

9. Baugruppe zum Sammeln von Harnsteinen, umfassend:
eine Sammelvorrichtung nach einem der Ansprüche 1 bis 8,
Urin-pH-Wert-Teststreifen,
ein Formular zum Identifizieren und zum Aufzeichnen eines Urin-pH-Wert-Testergebnisses, und
eine Hülle für die Weitergabe der Sammelvorrichtung und des Identifizierungs- und Aufzeichnungsformulars.

10. Baugruppe nach Anspruch 9, umfassend eine Tasche mit einem Verschluss zum Empfangen und Einschließen der Sammelvorrichtung auf wasserdichte Weise.

## Claims

1. A urinary stone collecting device comprising:
a filter part (1a, 11a, 21a) having a concave face and an opposite convex face, and a multiplicity of holes forming a filter (2, 12, 22),
a lid part (1b, 11b, 21b) connected to the filter part by a hinge (5, 15, 25a), so as to be movable between a deployed position where it acts as a handle, and a closed position where it covers the concave face of the filter part and encloses a volume between the filter and lid parts, in order to retain urinary stones collected by the filter, and
a locking device (4a, 4b, 14a, 14b, 24a, 24b) for the filter and lid parts in the closed position,
**characterized in that** the filter part (1a, 11a, 21a) comprises a receptacle (3, 13, 23) for collecting urine for performing a urine pH analysis.

2. The device of claim 1, wherein the hinge (5, 25a) has a hinge axis oriented parallel to an opening plane of the filter part (1a, 21a).

3. The device of any of claims 1 and 2, wherein the lid part (1b, 11b, 21b) has a generally similar shape to the filter portion (1a, 11a, 21a).

4. The device of claim 1, wherein the hinge (15) has a hinge axis oriented perpendicular to an opening plane of the filter part (11a).

5. The device according to claim 4, wherein the lid part (1b, 11b, 21b) has a concave face and a convex face, the convex face of the lid being arranged in the closed position opposite the filter part (1a, 11a, 21a), so as to ensure the locking of the filter and lid parts in the closed position.

6. The device according to one of claims 1 to 5, comprising a flap (21c) covering the concave face of the filter part (21a), the flap being connected by a hinge (25b) to the filter part and/or to the lid part (21b), so as to be movable between a closed position in which it cooperates with the lid part to form a closed volume housing the filter part, and a deployed position in which it cooperates with the lid part in order to serve as a handle for holding the filter part.

7. The device according to one of claims 1 to 5, wherein the lid part comprises two side portions pivotally connected to the filter part, so as to be movable between a closed position where they form a closed volume housing the filter part, and a deployed position where they cooperate together to form a handle for holding the filter part.

8. The device according to one of claims 1 to 7, wherein the lid part (21b) forms a housing for receiving a roll (27) of urine pH test strips.

9. A urinary stone collection assembly comprising:
a collection device according to any of claims 1 to 8,
urine pH test strips,
a sheet for identifying and recording a urine pH test result, and
an envelope for transmitting the collection device and the identification and recording sheet.

10. The assembly of claim 9, comprising a pocket with a closure for receiving and sealing the collection device
